# FASCICULE DE BREVET EUROPEEN

(11) **EP 2 197 522 B1**
(45) Date de publication et mention de la délivrance du brevet: **10.06.2015**
(21) Numéro de dépôt: 07858403.4
(22) Date de dépôt: 05.10.2007
(51) Int. Cl.: A61M 5/20, A61M 5/315

(54) **DISPOSITIF D'INJECTION AUTOMATIQUE D'UN PRODUIT DANS UN SITE D'INJECTION**
VORRICHTUNG ZUR AUTOMATISCHEN INJEKTION EINES PRODUKTS IN EINE INJEKTIONSSTELLE
DEVICE FOR THE AUTOMATIC INJECTION OF A PRODUCT IN AN INJECTION SITE

(43) Date de publication de la demande: 23.06.2010
(73) Titulaire: Becton Dickinson France, 38800 Le Pont-de-Claix (FR)
(72) Inventeur: ABRY, Hervé, 38800 Champagnier (FR); MARITAN, Lionel, 38119 Pierre-Chatel (FR); PEROT, Frédéric, 38760 Saint-Paul De Varces (FR); CARREL, Frank, 38220 Saint Jean de Vaulx (FR)
(74) Mandataire: Jeannet, Olivier
(86) Numéro de dépôt international: PCT/FR2007/001634
(87) Numéro de publication internationale: WO 2009/043979

(56) Documents cités:
- WO-A-02/47746
- WO-A-2007/036676
- FR-A- 2 654 938
- US-A1- 2005 101 919
- US-A1- 2006 229 570

## Description

La présente invention concerne un dispositif d'injection automatique d'un produit dans un site d'injection.

Dans la description qui suit, les termes "proximal" et "distal" sont à considérer par rapport à la main de l'utilisateur ayant saisi le dispositif, "proximal" se rapportant à une zone proche de cette main et "distal" à une zone éloignée de cette main. De la même manière, le terme "direction distale" veut dire la direction de l'injection, et le terme "direction proximale" veut dire la direction opposée à la direction d'injection.

Un dispositif d'injection automatique est un dispositif permettant à un utilisateur de s'injecter lui-même un produit. De manière connue en soi, un tel dispositif comprend :
- un boîtier propre à recevoir un récipient contenant le produit et équipé d'une aiguille, ce récipient étant déplaçable par rapport au boîtier entre une position initiale et une position d'insertion, distante dans le sens distal par rapport à la position initiale ;
- un organe de protection relié au boîtier et déplaçable par rapport à ce boîtier vers une première position dans laquelle un mouvement du récipient de sa position initiale à sa position d'insertion est possible et une deuxième position, espacée par rapport à la première position, l'organe de protection ayant une extrémité libre qui est située au-delà d'une extrémité distale de l'aiguille lorsque l'organe de protection est dans ladite deuxième position ;
- des moyens d'activation, sélectivement déplaçables d'une première position vers une deuxième position dans laquelle le récipient est apte à être déplacé de sa position initiale à sa position d'insertion ;
- des premiers et deuxièmes moyens de verrouillage pour verrouiller lesdits moyens d'activation.

L'ensemble est tel que, dans ladite deuxième position (deuxième position) de l'organe de protection, lesdits premiers et deuxièmes moyens de verrouillage immobilisent les moyens d'activation, prévenant ainsi le risque de mauvaise manipulation pouvant conduire à une perte de produit. Les moyens d'activation sont libérés lorsque l'organe de protection a été suffisamment appliqué contre la peau pour être amené dans ladite première position (première position).

En pratique, l'utilisateur saisit le dispositif par le boîtier et applique l'organe de protection contre sa peau de manière à faire passer cet organe de protection de la deuxième position à la première position. Ce passage rend possible le passage du récipient, et donc de l'aiguille, de la position initiale à la position d'insertion, et permet de libérer le mouvement des moyens d'activation. L'utilisateur déplace ensuite ces moyens d'activation pour réaliser l'injection.

Les demandes de brevet français N° FR 06 07806 et N° FR 06 03200, au nom de la demanderesse, illustrent des dispositifs d'injection automatique connus. Les documents WO 02/47746, US2005/0101919, WO 2007/036676 et FR 2.654.938 décrivent d'autres dispositifs d'injection automatique.

Les dispositifs connus permettent de sécuriser une injection automatique dans une certaine mesure mais n'excluent pas tout risque de mauvaise manipulation. En effet, les utilisateurs peuvent être plus ou moins stressés par la crainte que génère l'injection et la douleur qu'ils redoutent, et risquent soit de retirer plus ou moins le dispositif après avoir commencé à appuyer sur les moyens d'activation, de sorte que l'aiguille n'est pas assez enfoncée dans la peau, soit de décider au dernier moment de changer l'emplacement d'injection. Il peut en résulter un actionnement défectueux du dispositif, une blessure par l'aiguille d'injection et/ou une perte plus ou moins importante de produit.

La présente invention a pour objectif principal de remédier à cet inconvénient, en fournissant un dispositif d'injection ayant une sécurité renforcée, réduisant fortement le risque de mauvaise manipulation au moment de l'injection.

Un autre objectif que l'invention est de fournir un dispositif conservant une structure relativement simple et un prix de fabrication acceptable.

Le dispositif d'injection automatique concerné comprend, de manière connue en soi :
- un boîtier propre à recevoir un récipient contenant le produit et équipé d'une aiguille, ce récipient étant déplaçable par rapport au boîtier entre une position initiale et une position d'insertion, distante dans le sens distal par rapport à la position initiale ;
- un organe de protection relié au boîtier et déplaçable par rapport à ce boîtier vers une première position dans laquelle un mouvement du récipient de sa position initiale à sa position d'insertion est possible et une deuxième position, espacée par rapport à la première position, l'organe de protection ayant une extrémité libre qui est située au-delà d'une extrémité distale de l'aiguille lorsque l'organe de protection est dans ladite deuxième position ;
- des moyens d'activation, sélectivement déplaçables d'une première position vers une deuxième position dans laquelle le récipient est apte à être déplacé de sa position initiale à sa position d'insertion ;
- des premiers et deuxièmes moyens de verrouillage pour verrouiller lesdits moyens d'activation.

Selon l'invention,
- les premiers et deuxièmes moyens de verrouillage sont conformés pour venir en prise les uns avec les autres et permettre le verrouillage des moyens d'activation dans au moins une position intermédiaire entre lesdites première et deuxième positions des moyens d'activation ;
- les premiers et deuxièmes moyens de verrouillage sont conformés pour être hors de prise les uns d'avec les autres lorsque l'organe de protection est dans ladite première position et pour venir en prise les uns avec les autres lorsque l'organe de protection est en dehors de ladite première position.

Ainsi, selon l'invention, le dispositif comprend des moyens de verrouillage permettant un verrouillage des moyens d'activation dans au moins une position intermédiaire, et qui sont amenés en position de déverrouillage uniquement lorsque l'organe de protection est dans ladite première position. Par conséquent, la perte de cette première position va conduire à un retour des moyens de verrouillage en coopération mutuelle même si les moyens d'activation se trouvent dans ladite au moins une position intermédiaire, ce retour amenant à un re-verrouillage des moyens d'activation.

Ce re-verrouillage prévient les risques de mauvaises manipulations liées à une perte de la première position alors que les moyens d'activation ont déjà été partiellement déplacés et qu'ils se trouvent dans ladite au moins une position intermédiaire.

Le dispositif selon l'invention présente donc une sécurité renforcée à l'égard d'une manipulation défectueuse.

Les moyens d'activation peuvent commander uniquement l'injection ; dans ce cas, l'insertion de l'aiguille dans la peau du patient se fait lors du mouvement de l'organe de protection de ladite deuxième position à ladite première position, ce mouvement étant tel que l'extrémité distale de l'organe de protection vient, au cours de ce mouvement, en deçà de l'extrémité libre de l'aiguille, rendant ainsi possible la pénétration de cette aiguille dans la peau du patient.

Les moyens d'activation peuvent également commander à la fois l'insertion de l'aiguille dans la peau du patient et l'injection ; dans ce cas, dans ladite première position, l'extrémité distale de l'organe de protection se situe toujours au-delà de l'extrémité libre de l'aiguille, et c'est l'actionnement des moyens d'activation qui commande la pénétration de l'aiguille.

Avantageusement, lesdits premiers et deuxièmes moyens de verrouillage sont conformés pour permettre le verrouillage des moyens d'activation dans une position intermédiaire faisant partie d'une pluralité de positions successives intermédiaires entre lesdites première et deuxième positions des moyens d'activation.

Le re-verrouillage des moyens d'activation est ainsi possible selon une pluralité de positions intermédiaires.

Lesdits premiers et deuxièmes moyens de verrouillage peuvent notamment comprendre des crans complémentaires aménagés sur lesdits moyens d'activation et sur une pièce intermédiaire d'actionnement, ces crans étant propres à venir réciproquement en prise.

Selon une forme de réalisation préférée de l'invention, ladite pièce intermédiaire d'actionnement inclut une base destinée à venir en prise, de manière déflectable, avec l'organe de protection lorsque cet organe de protection est dans ladite première position.

Avantageusement, l'organe de protection est verrouillable dans ladite deuxième position.

De préférence, lesdits premier et deuxième moyens de verrouillage viennent réciproquement en prise et verrouillent lesdits moyens d'activation dans une position intermédiaire entre lesdites première et seconde positions si l'organe de protection se déplace en dehors de sa première position avant un déplacement prédéterminé des moyens d'activation.

Ce déplacement prédéterminé est de préférence suffisant pour amener le récipient à se déplacer jusque dans ladite position d'insertion.

L'invention sera bien comprise, et d'autres caractéristiques et avantages de celle-ci apparaîtront, en référence au dessin schématique annexé, représentant, à titre d'exemple non limitatif, une forme de réalisation préférée du dispositif d'injection automatique qu'elle concerne.

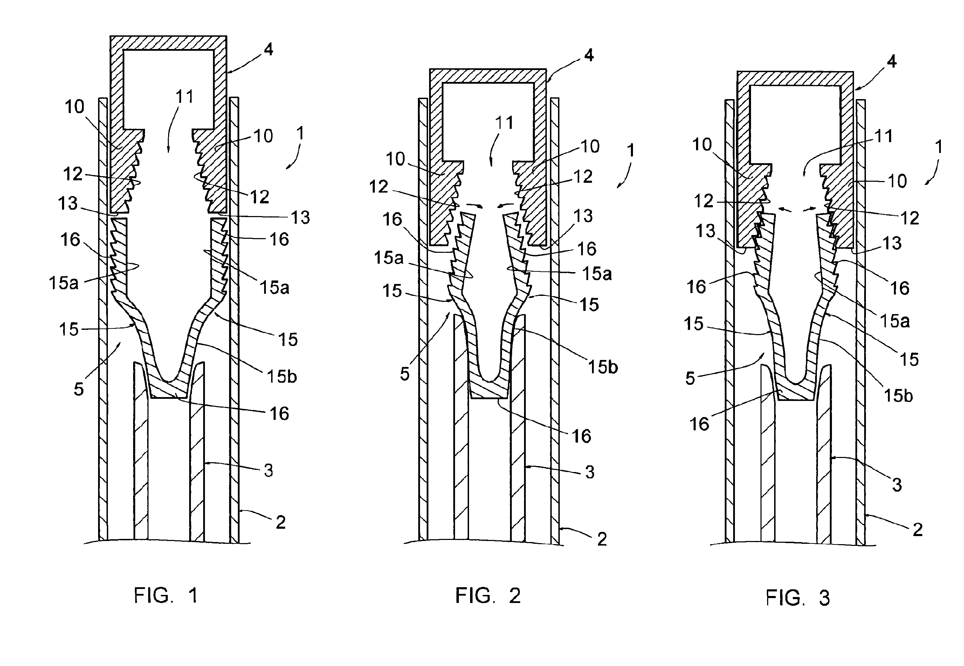

Les figures 1 à 3 sont des vues schématiques, très simplifiées, de ce dispositif, en coupe passant par son axe longitudinal, au cours de trois positions d'utilisation, à savoir respectivement une position de repos, une position intermédiaire permettant d'insertion de l'aiguille et l'injection, et une position de re-verrouillage d'un bouton proximal d'actionnement qu'il comprend dans cette position intermédiaire.

Les figures représentent un dispositif d'injection automatique 1, comprenant :
- un corps de seringue, une aiguille creuse d'injection et un piston engagé dans le corps de seringue (non représentés) ;
- un boîtier 2 ;
- un tube de protection 3 ;
- un ressort (non représenté) maintenant normalement le tube de protection 3 dans la position de repos (dite "deuxième position") montrée sur la figure 1 ;
- un bouton proximal 4 d'actionnement, permettant de commander le déplacement du corps de seringue d'une position initiale à une position d'insertion de l'aiguille et/ou de commander l'injection, et
- une pièce intermédiaire 5 d'actionnement.

Le boîtier 2 est destiné à être saisi par l'utilisateur au moment de l'injection.

Le tube de protection 3 est engagé autour du corps de seringue et de l'aiguille d'injection, et est engagé dans le boîtier 2 en étant mobile en coulissement par rapport à celui-ci. Cette mobilité se fait entre ladite deuxième position (cf. figure 1), dans laquelle l'extrémité distale du tube de protection 3 se situe au-delà, dans le sens distal, de l'extrémité distale de l'aiguille d'injection, protégeant l'utilisateur contre tout risque de blessure pouvant être occasionné par cette aiguille, et une position d'utilisation, dite "première position" (cf. figure 2), dans laquelle l'extrémité distale du tube 3 se situe en-deçà, dans le sens distal, de l'extrémité distale de l'aiguille d'injection. Le dispositif 1 comprend des moyens de limitation du coulissement du tube 3 par rapport au corps 2, agissant de telle sorte que le retrait du tube 3 par rapport à l'extrémité distale de l'aiguille soit telle que ce retrait corresponde à la profondeur idéale de pénétration de l'aiguille dans la peau du patient.

Il apparaît sur les figures que le tube de protection 3 présente, à son extrémité proximale, une portion de forme légèrement évasée et un rebord proximal interne arrondi.

Le bouton d'activation 4 comprend une partie (non représentée) permettant de commander le déplacement du corps de seringue de ladite position initiale à ladite position d'insertion. Du côté distal, il se divise en deux parties 10 diamétralement opposées, dont les faces en regard sont inclinées par rapport à l'axe longitudinal du dispositif 1 et délimitent entre elles un espace vide 11. Ces faces inclinées présentent chacune une série de crans 12. En outre, chaque partie 10 se termine par une face plane 13 orientée perpendiculairement à l'axe longitudinal du bouton 4.

La pièce intermédiaire d'actionnement 5 présente une forme générale en "V" ou en "Y", c'est-à-dire comprend deux branches latérales 15 diamétralement opposées et une partie de base 16 destinée à être engagée dans le tube de protection 3.

Chacune de ces branches latérales 15 comprend, du côté du bouton 4 une portion d'extrémité 15a rectiligne, pourvue de crans 16 sur sa face radialement externe, et, du côté du tube 3, une portion de base 15b arrondie, s'évasant dans le sens proximal.

Les deux portions de base 15b sont raccordées de manière continue à la partie de base 16, laquelle est, en deuxième position, engagée dans l'extrémité proximale du tube de protection 3, ainsi que le montre la figure 1.

L'ensemble de cette pièce 5 est réalisé par moulage d'une matière synthétique présentant un degré de déformation élastique. En position normale de non déformation, la pièce 5 est telle que montrée sur la figure 1, avec ses branches latérales 15 éloignées l'une de l'autre et les extrémités proximales de ces branches 15 venant en regard des faces planes distales 12 du bouton 4 et réalisant ainsi un verrouillage du déplacement axial de ce dernier. La pièce 5 peut être déformée élastiquement jusqu'à la position montrée sur la figure 2, dans laquelle les branches latérales 15 sont rapprochées l'une de l'autre jusqu'à venir au-delà des faces planes distales 12. Le bouton 4 peut alors être engagé sur les portions proximales 15a de ces branches latérales 15 grâce à l'espace vide 11 délimité par les deux portions 10, de sorte que le déplacement de ce bouton 4 est libéré, permettant, par une pression sur la face proximale de ce bouton, de réaliser l'insertion de l'aiguille et/ou l'injection.

Il apparaît sur la figure 2 que la déformation de la pièce 5 résulte de la venue du tube de protection 3 dans ladite première position, ce tube 3 venant porter contre les portions distales arrondies 15b des branches 15, qui constituent ainsi des rampes de commande du mouvement de rapprochement des portions d'extrémité 15a en rapprochement l'une de l'autre.

Dans le cadre de l'utilisation normale du dispositif 1, le bouton 4 peut être pressé dans la direction distale jusqu'à ce que sa face proximale vienne affleurer avec l'extrémité proximale du boîtier 2.

Cependant, si le tube de protection 3 venait à quitter ladite première position montrée sur la figure 2, et donc était ramené par le ressort précité dans ladite deuxième position montrée sur la figure 1, il apparaît sur la figure 3 que les deux branches 15 seraient immédiatement ramenées dans des positions d'éloignement mutuel sous l'effet du rappel élastique du matériau constituant la pièce 5. Cet éloignement conduit, selon la position axiale du bouton 4, au moins deux crans 16, ou plus de deux crans 16, à venir en prise avec deux crans 12, ou plus de deux crans 12, du bouton 4. La venue en prise de ces crans 16, 12 permet d'immobiliser à nouveau le bouton 4 axialement, et donc d'empêcher une poursuite de l'injection tant que le tube de protection 3 n'est pas revenu dans ladite première position.

Il doit être noté que la pluralité de crans et la pluralité de crans permettent le verrouillage du bouton d'activation 4 dans une pluralité de positions intermédiaires de déplacement successives.

Comme cela apparaît de ce qui précède, l'invention fournit un dispositif d'injection automatique présentant, par rapport aux dispositifs homologues de la technique antérieure, les avantages déterminants d'avoir une sécurité renforcée, réduisant fortement le risque de mauvaise manipulation au moment de l'injection, et de conserver une structure relativement simple et un prix de fabrication acceptable.

L'invention a été décrite ci-dessus en référence à une forme de réalisation donnée à titre de pur exemple. Il va de soi qu'elle n'est pas limitée à cette forme de réalisation mais qu'elle s'étend à toutes les formes de réalisations couvertes par les revendications ci-annexées.

## Revendications

1. Dispositif (1) d'injection automatique d'un produit dans un site d'injection, comprenant :
- un boîtier (2) propre à recevoir un récipient contenant le produit et équipé d'une aiguille, ce récipient étant déplaçable par rapport au boîtier (2) entre une position initiale et une position d'insertion, distante dans le sens distal par rapport à la position initiale ;
- un organe de protection (3) relié au boîtier (2) et déplaçable par rapport à ce boîtier (2) vers une première position dans laquelle un mouvement du récipient de sa position initiale à sa position d'insertion est possible et vers une deuxième position, dans laquelle l'organe de protection (3) est verrouillé, l'organe de protection (3) ayant une extrémité libre qui est située au-delà d'une extrémité distale de l'aiguille lorsque l'organe de protection (3) est dans ladite deuxième position ;
- des moyens d'activation (4), sélectivement déplaçables d'une première position vers une deuxième position dans laquelle le récipient est apte à être déplacé de sa position initiale à sa position d'insertion ;
- des premiers et deuxièmes moyens (16 ; 12) de verrouillage pour verrouiller lesdits moyens d'activation (4) ;
**caractérisé en ce que** :
- les premiers et deuxièmes moyens de verrouillage (16 ; 12) sont conformés pour venir en prise les uns avec les autres et permettre le verrouillage des moyens d'activation (4) dans au moins une position intermédiaire entre lesdites première et deuxième positions des moyens d'activation (4) ;
- les premiers et deuxièmes moyens de verrouillage (16 ; 12) sont conformés pour être hors de prise les uns d'avec les autres lorsque l'organe de protection (3) est dans ladite première position et pour venir en prise les uns avec les autres lorsque l'organe de protection (3) est en dehors de ladite première position.

2. Dispositif (1) selon la revendication 1, **caractérisé en ce que** lesdits premiers et deuxièmes moyens de verrouillage (16 ; 12) sont conformés pour permettre le verrouillage des moyens d'activation (4) dans une position intermédiaire faisant partie d'une pluralité de positions successives intermédiaires entre lesdites première et deuxième positions des moyens d'activation (4).

3. Dispositif (1) selon la revendication 1 ou la revendication 2, **caractérisé en ce que** lesdits premiers et deuxièmes moyens de verrouillage comprennent des crans (16 ; 12) complémentaires aménagés sur lesdits moyens d'activation (4) et sur une pièce intermédiaire d'actionnement (5), ces crans (16 ; 12) étant propres à venir réciproquement en prise.

4. Dispositif (1) selon la revendication 3, **caractérisé en ce que** ladite pièce intermédiaire d'actionnement (5) inclut une base (16) destinée à venir en prise, de manière déflectable, avec l'organe de protection (3) lorsque cet organe de protection (3) est dans ladite première position.

5. Dispositif (1) selon l'une des revendications 1 à 4, **caractérisé en ce que** lesdits premier et deuxième moyens de verrouillage (16, 12) viennent réciproquement en prise et verrouillent lesdits moyens d'activation (4) dans une position intermédiaire entre lesdites première et seconde positions si l'organe de protection (3) se déplace en dehors de sa première position avant un déplacement prédéterminé des moyens d'activation (4).

6. Dispositif (1) selon la revendication 5, **caractérisé en ce que** le déplacement prédéterminé des moyens d'activation est suffisant pour amener le récipient à se déplacer jusque dans ladite position d'insertion.

## Patentansprüche

1. Vorrichtung (1) zur automatischen Injektion eines Produkts in eine Injektionsstelle, umfassend:
- ein Gehäuse (2), das geeignet ist, einen Behälter aufzunehmen, der das Produkt enthält und mit einer Nadel ausgerüstet ist, wobei dieser Behälter in Bezug zum Gehäuse (2) zwischen einer Ausgangsstellung und einer Einsatzstellung verschiebbar ist, die in distaler Richtung von der Ausgangsstellung entfernt ist;
- ein Schutzorgan (3), das mit dem Gehäuse (2) verbunden ist und in Bezug zu diesem Gehäuse (2) in Richtung einer ersten Stellung, in der eine Bewegung des Behälters von seiner Ausgangsstellung in seine Einsatzstellung möglich ist, und in Richtung einer zweiten Stellung verschoben werden kann, in der das Schutzorgan (3) verriegelt ist, wobei das Schutzorgan (3) ein freies Ende aufweist, das sich jenseits eines distalen Endes der Nadel befindet, wenn das Schutzorgan (3) sich in der zweiten Stellung befindet;
- Aktivierungsmittel (4), die selektiv von einer ersten Stellung in Richtung einer zweiten Stellung verschiebbar sind, in der der Behälter geeignet ist, von seiner Ausgangsstellung in seine Einsatzstellung verschoben zu werden;
- erste und zweite Verriegelungsmittel (16; 12) zum Verriegeln der Aktivierungsmittel (4);
**dadurch gekennzeichnet, dass**:
- die ersten und zweiten Verriegelungsmittel (16; 12) angepasst sind, miteinander in Eingriff zu gelangen und das Verriegeln der Aktivierungsmittel (4) in mindestens einer Zwischenstellung zwischen der ersten und der zweiten Stellung der Aktivierungsmittel (4) zu ermöglichen;
- die ersten und zweiten Verriegelungsmittel (16; 12) angepasst sind, miteinander außer Eingriff gebracht zu werden, wenn das Schutzorgan (3) sich in der ersten Stellung befindet, und miteinander in Eingriff zu gelangen, wenn das Schutzorgan (3) sich außerhalb der ersten Stellung befindet.

2. Vorrichtung (1) nach Anspruch 1, **dadurch gekennzeichnet, dass** die ersten und zweiten Verriegelungsmittel (16; 12) angepasst sind, das Verriegeln der Aktivierungsmittel (4) in einer Zwischenstellung zu ermöglichen, die Teil mehrerer aufeinanderfolgender Zwischenstellungen zwischen der ersten und der zweiten Stellung der Aktivierungsmittel (4) ist.

3. Vorrichtung (1) nach Anspruch 1 oder Anspruch 2, **dadurch gekennzeichnet, dass** die ersten und zweiten Verriegelungsmittel ergänzende Rastkerben (16; 12) aufweisen, die auf den Aktivierungsmitteln (4) und auf einem Betätigungszwischenteil (5) eingerichtet sind, wobei diese Rastkerben (16; 12) geeignet sind, gegenseitig in Eingriff zu gelangen.

4. Vorrichtung (1) nach Anspruch 3, **dadurch gekennzeichnet, dass** das Betätigungszwischenteil (5) eine Basis (16) aufweist, die dazu bestimmt ist, auf ablenkbare Weise mit dem Schutzorgan (3) in Eingriff zu gelangen, wenn dieses Schutzorgan (3) sich in der ersten Stellung befindet.

5. Vorrichtung (1) nach einem der Ansprüche 1 bis 4, **dadurch gekennzeichnet, dass** das erste und zweite Verriegelungsmittel (16, 12) gegenseitig in Eingriff gelangen und die Aktivierungsmittel (4) in einer Zwischenstellung zwischen der ersten und der zweiten Stellung verriegeln, wenn das Schutzorgan (3) sich vor einer vorbestimmten Verschiebung der Aktivierungsmittel (4) aus seiner ersten Stellung heraus bewegt.

6. Vorrichtung (1) nach Anspruch 5, **dadurch gekennzeichnet, dass** die vorbestimmte Verschiebung der Aktivierungsmittel ausreicht, um den Behälter dazu zu bringen, sich bis in die Einsatzstellung zu bewegen.

## Claims

1. A device (1) for automatic injection of a product into an injection site, comprising:
- a housing (2) capable of receiving a container containing the product and equipped with a needle, this container being movable relative to the housing (2) between an initial position and an insertion position, distally spaced relative to the initial position, and
- a safety shield member (3) coupled to and movable with respect to the housing (2) to a first position in which movement of the container from its initial position to its insertion position is possible, and to a second position in which the safety shield member (3) is locked, the safety shield member (3) having a free end that is distally spaced beyond a distal end of the needle when the safety shield member (3) is in the second position,
- activating means (4) selectively movable from a first position to a second position in which the container is able to be moved from its initial position to its insertion position;
- first and second locking means (16; 12) for locking said activating means (4);
**characterized in that**:
- the first and second locking means (16; 12) are formed to engage each other and allow the locking of the activating means (4) in at least one position intermediate the first and second position of the activating means (4);
- the first and second locking means (16; 12) being formed to disengage from each other when the safety shield member (3) is in said first position, and to engage each other when the safety shield member (3) is out of the first position.

2. Device (1) according to claim 1, **characterized in that** the aforementioned first and second locking means (16; 12) are formed to allow the locking of the activating means (4) in one of a plurality of successive positions intermediate the first and second position of the activating means (4).

3. Device (1) according to claim 1 or claim 2, **characterized in that** the aforementioned first and second locking means include complementary locks (16; 12) on the activating means (4) and on an intermediary actuation member (5), the complementary locks (16; 12) being engageable with each other.

4. Device (1) according to claim 3, **characterized in that** the aforementioned intermediary actuation member (5) includes a base (16) intended to be deflectably engaged with the safety shield member (3) when the safety shield member (3) is in said first position.

5. Device (1) according to one of claims 1 to 4, **characterized in that** the aforementioned first and second locking means (16; 12) engage each other and lock the activating means (4) in a position intermediate the first and second position if the safety shield member (3) is moved out of its first position prior to a predetermined displacement of the activating means (4).

6. Device (1) according to claim 5, **characterized in that** the predetermined displacement of the activating means is sufficient to cause the container to move to said insertion position.
